Europäisches Patentamt

European Patent Office

Office européen des brevets

(19)

(11) Numéro de publication: **0 226 513**
**A1**

(12) **DEMANDE DE BREVET EUROPEEN**

(21) Numéro de dépôt: **86402717.2**

(22) Date de dépôt: **08.12.86**

(51) Int. Cl.4: **C 07 K 5/00**
C 07 K 7/00, A 61 K 37/64,
G 01 N 33/569, C 12 P 21/02

(30) Priorité: **06.12.85 FR 8518155**

(43) Date de publication de la demande:
**24.06.87 Bulletin 87/26**

(84) Etats contractants désignés:
**AT BE CH DE ES FR GB GR IT LI LU NL SE**

(71) Demandeur: **INSTITUT PASTEUR**
**25-28, rue du Docteur Roux**
**F-75724 Paris Cédex 15 (FR)**

(72) Inventeur: **Auffray, Charles**
**30, rue des Moissonneurs**
**F-94440 Marolles en Brie (FR)**

**Montagnier, Luc**
**21, rue de Malabry**
**F-92350 Le Plessis Robinson (FR)**

**Klatzmann, David**
**84, Quai de Jemmapes**
**F-75011 Paris (FR)**

**Fisher, Alain**
**15, passage Trubert-Béllier**
**F-75013 Paris (FR)**

**Charron, Dominique**
**10, rue de Reims**
**F-75013 Paris (FR)**

(74) Mandataire: **Gutmann, Ernest et al**
**S.C. Ernest Gutmann - Yves Plasseraud 67, boulevard**
**Haussmann**
**F-75008 Paris (FR)**

(54) Peptides capables d'inhiber les interactions entre des antigènes et les lymphocytes T4, produits qui en sont dérivés et leurs applications.

(57) L'invention concerne un polypeptide ayant la capacité d'inhiber l'interaction du virus tenu pour responsable du SIDA avec les récepteurs T4 portés par les lymphocytes T4. Ce peptide est choisi parmi ceux qui sont représenté parmi les formules générales :

X — — — — — — R F D S — — — — — — Z
X — — — — — — S D F R — — — — — — Z
X — — — — — — S D A R — — — — — — Z
X — — — — — — R A D S — — — — — — Z

dans lesquelles X est un atome d'hydrogène ou un groupe peptidique. Z est OH ou un groupe peptidique, et les autres lettres désignent des aminoacides spécifiques (nomenclature de désignation à une lettre des aminoacides).

EP 0 226 513 A1

## Description

Peptides capables d'inhiber les interactions entre des antigènes et les lymphocytes T4. produits qui en sont
dérivés et leurs applications

L'invention concerne des peptides dérivés des antigènes de HLA de classe II. de l'antigène T4 et de protéines HIV capables d'inhiber les coopérations cellulaires entre les macrophages. les lymphocytes et les lymphocytes B.

L'invention concerne en particulier des peptides ayant la capacité d'inhiber l'interaction du virus tenu pour responsable du SIDA (syndrome d'immunodéficience acquise) avec les récepteurs T4 portés par les lymphocytes T4 et. plus généralement d'exercer un effet immunomodulateur à l'égard des interactions entre les macrophages et les lymphocytes T auxiliaires. d'une part. et ces derniers et les lymphocytes B. d'autre part. dès lors que ces interactions sont normalement médiées par des récepteurs lymphocytaires codés par des complexes majeurs d'autohistocompatibilité de classe II (antigènes HLA de classe II chez l'homme). En d'autres termes. l'invention concerne plus particulièrement des peptides capables de moduler les réponses cellulaires mettant plus particulièrement en jeu des antigènes susceptibles d'induire des réponses immunitaires normalement médiées par des antigènes HLA de classe II. par exemple du type HLA DR.

Certains virus lymphotropes interfèrent avec ces réponses cellulaires, en particulier des virus tenus pour responsables du SIDA.

De tels virus ont été isolés par plusieurs équipes. Ils ont été désignés sous des abréviations diverses : LAV, HTLV-III. ARV2. etc.. Ces virus, dénommés ci-après sous l'abréviation la plus générale HIV (anciennement LAV/HTLV III). possèdent en effet un tropisme spécifique pour les lymphocytes T4. L'accès du virus aux lymphocytes T4 peut cependant être bloqué in vitro, lorsque ces derniers ont au préalable été incubés en présence d'anticorps monoclonaux (par exemple ceux commercialisés par la firme ORTHO sous la désignation OKT4) qui bloquent les récepteurs T4 de ces lymphocytes. C'est par référence à la capacité de ces anticorps monoclonaux de bloquer ces sites T4, qu'il sera fait plus particulièrement référence dans ce qui suit. à l'affinité des virus LAV pour les récepteurs T4 des lymphocytes T4.

Les antigènes mettant en jeu des antigènes inducteurs de réponses immunitaires relevant des antigènes HLA de classe II ne sont évidemment pas limités à ceux présents dans des virus HIV. On mentionnera. à titre d'exemple de ces autres antigènes les virus de la grippe (influenza), d'Epstein-Barr, des rhinovirus, etc...

L'invention concerne également diverses applications de ces peptides et des produits qui peuvent en être dérivés. Par "produits dérivés de ces peptides". on entend des produits reconnus immunologiquement par des anticorps qui reconnaissent également ces peptides ou. encore. des produits susceptibles d'exercer sensiblement les mêmes effets au niveau des susdites médiations cellulaires.

A titre d'exemples d'applications de ces peptides, on peut mentionner le diagnostic. voire le traitement ou la prévention des maladies contrôlées par les interactions entre les molécules HLA de classe II et l'antigène T4, parmi lesquelles les maladies induites par HIV.

D'autres applications de ces peptides peuvent s'adresser à la détection de ceux des antigènes qui sont susceptibles d'induire des réactions immunitaires relevant de certaines catégories d'antigènes HLA de classe II. par exemple de HLA-DR.

D'autres applications de ces peptides s'adressent. en variante, et lorsque le type d'antigène HLA susceptible de médier les réactions immunitaires induites par un antigène déterminé, à leur utilisation dans des tests visant à étudier in vitro l'effet immunomodulateur de substances à l'étude.

L'invention découle de l'hypothèse initiale faite par les inventeurs que l'infection des lymphocytes T4 par les virus LAV et le pouvoir pathogène de ces derniers tenaient à leur capacité d'interférer avec des séquences peptidiques concernées appartenant aux antigènes régulant les capacités de reconnaissance par ces lymphocytes de cellules porteuses de ces antigènes viraux, lorsque ceuxci leur sont présentés par les macrophages, par l'intermédiaire des récepteurs lymphocytaires appropriés. Parmi ces antigènes, on mentionnera plus particulièrement ceux qui sont constitués par des complexes majeurs d'auto-histocompatibilité, connus sous l'expression anglaise "self major histocompatibility complexes" et l'abréviation correspondante MHC, qui sera souvent utilisée dans ce qui suit. Les antigènes codés par le MHC sont appelés HLA chez l'homme.

Les fonctions des divers types de lymphocytes T4 ou T8 impliquent des interactions avec les molécules codées par le système MHC de classe II pour les T4 et de classe I pour les T8.

Leurs interactions respectives paraissent médiées par des molécules auxiliaires, parmi lesquelles des antigènes de différentiation T-cellulaires (notamment antigènes T4 et T8) et des molécules qui ont été décrites comme facteurs potentiels d'adhésion aux cellules. Pour plus de détails, on peut se reporter aux publications de (11). (12) et (13) identifiées à la fin de la présente description.

Les recherches qui ont abouti à la présente invention ont été fondées sur la recherche de séquences peptidiques qui pourraient être tenues pour responsables des capacités d'adhésion mutuelle. d'une part des lymphocytes T4 et, d'autre part. soit des antigènes MHC de classe II qui sont susceptibles de les reconnaître, soit des virus LAV, dont on présuppose qu'ils pourraient "mimer" l'action desdits antigènes vis-à-vis desdits lymphocytes.

Ces hypothèses ayant été posées. les inventeurs ont mis en évidence l'existence de séquences peptidiques communes aux chaînes $\alpha$ de nombreux antigènes MHC de classe I. aux chaînes $\beta$ des antigènes de classe II et à une protéine codable par l'une des régions de lecture ouverte que l'on retrouve dans les génomes des différentes espèces de souches LAV qui ont été isolées. La mise en évidence de cette séquence commune, consistant en une séquence tétrapeptidique Arg-Phe-Asp-ser est d'autant plus remarquable qu'elle se trouve dans la seule région d'homologie connue à ce jour dans nombres des antigènes des classes I et II dont les séquences polypeptidiques partielles ou totales ont été analysées (18) et (19).

Dans ce qui suit, on fera souvent appel à la nomenclature officielle relative aux désignations des aminoacides naturels par une lettre unique, nomenclature que l'on rappelle ci-dessous.

M méthionine
L leucine
I isoleucine
V valine
F phénylalanine
S sérine
P proline
T thréonine
A alanine
Y tyrosine
H histidine
Q glutamine
N asparagine
K lysine
D acide aspartique
E acide glutaminique
C cystéine
W tryptophane
R arginine
G glycine

En conséquence la séquence tétrapeptidique Arg-Phe-Asp-Ser est désignée dans la nomenclature susdite par RFDS.

Les séquences tétrapeptidiques codables par le génome de LAV sont corrélables à une phase de lecture ouverte présente dans la partie d'extrémité 3′ qui chevauche la "région de répétition longue" (de l'anglais "long terminal repeat") dans la région U3 du génome (20). Il a déjà été suggéré que parmi les protéines codées par la phase de lecture ouverte de la région d'extrémité 3′, il pouvait y en avoir une qui, soit consisterait en un régulateur de transcription (21), soit interviendrait dans la cytotoxicité à l'égard des cellules T (20). En particulier une protéine F (p27) a été mise en évidence par Allan et al dans Science, 15 novembre 1985, 230, p. 813. Cette protéine F contient la séquence RFDS.

Plus particulièrement encore, on observe un dipeptide commun EL situé dans la même région d'homologie dans de nombreux antigènes de classe II et dans la séquence peptidique codable concernée des virus LAV, et à la même distance (9 résidus aminoacide les séparent) de la séquence tétrapeptidique précédente du côté de l'extrémité C-terminale de celle-ci, tant dans les antigènes de classe II que dans lesdites séquences codables du génome de LAV. Ce dipeptide est inexistant dans les régions d'homologie correspondantes des antigènes de classe I.

De même, on observe fréquemment, du côté de l'extrémité N-terminale du tétrapeptide une séquence dipeptidique (RE) commune entre la susdite région d'homologie des antigènes de classe II et le polypeptide codable correspondant du génome des virus LAV, si ce n'est que dans la susdite région d'homologie, cette séquence dipeptidique est séparée de la séquence RFDS par 3 résidus aminoacide et dans le polypeptide codable du virus par 4 résidus aminoacide. Une séquence dipeptidique SL est également présente. Elle est séparée de RFDS par 13 acides aminés. Cette séquence pourrait jouer un rôle dans la fixation du virus HIV ou de la protéine F sur la protéine T4.

Les résultats des observations qui ont été faites résultent du tableau ci-après dans lequel ont été rapportées les parties des séquences peptidiques en cause, contenues dans les polypeptides initialement étudiés ou séquencés et appartenant aux antigènes des classes I et II décrites dans les publications (19), (26), (27), (28), (29), (30) et (31) ou codables par des régions de lecture ouverte des ADNs correspondants de différents types de virus LAV (20), (21), (22) et (23) dans lesquelles les

TABLEAU

```
              20  *           30               40        *      50
              .   .           .                .        .       .
       A3      P R F I A V G Y V D D T Q F V|R F D S|D A A S Q R M E P R A P
       A28     P R F I A V G Y V D D T Q F V|R F D S|D A A S Q R M E P R A P
       A2      P R F I A V G Y V D D T Q F V|R F D S|D A A S Q R M E P R A P
       Aw24    P R F I A V G Y V D D T Q R V|R F D S|D A A S Q R M E P R A P
HLA    B7      P R F S A V C Y V D D T Q F V|R F D S|D A A S P R E E P R A P
       B27     P R F T A V G Y V D D T L F V|R F D S|D A A S P R E E P R A P
       B40     P R F T A V G Y V D D T L F V|R F D S|D A T S P R M E P R A P
       Cw3     P H F I A V G Y V D D T L F V|R F D S|D D E S P R G E P R A P
       12.4    P R F I S V G Y V D D T Q F V|R F D S|D D A S P R E E P R A P

Classe I
         b
       Kb      P R Y M E V G Y V D D T E F V|R F D S|D A E N P R Y E P R A P
         d
       Kd      P R F I A V G Y V D D T Q F V|R F D S|D A D N P R F E P R A P
         b
       Db      P R F I S V G Y V D N K E F V|R F D S|D A E N P R Y E R R A P
         d
       Dd      P R Y M E V G Y V D N T E F V|R F D S|D A E N P R Y E P R A R
H-2      d
       Ld      P R F I S V G Y V D N K E F V|R F D S|D A E N P R Y E P R A P
       27.1    P W F I S V G Y V D D T Q F V|R F D S|D A E N P R M E P R A R
       Kw28    P R F I S V G Y V D D T E F V|R F D S|D A E N P R Y E P R A R
       Q10     P R F I I V G Y V D D T Q F V|R F D S|D A E T P R M E P R A P

Lapin  RLA       P R F I I V G Y V D D T Q F V|R F D S|D A A S P R M E Q R A P

       - - - - - - - - - - - - - - - - - - - - - - - - - - - - - - - - - - - - -

       DP2       Q R F L E R Y I Y N R E E F V|R F D S|D V G E F R A V T|E L|G
       DP3       Q R F L E R Y I Y N R E E F Y|R F D S|D V G E F R A V T|E L|G
       DP4       Q R F L E P Y I Y N R E E F A|R F D S|D V G E F R A V T|E L|G
       DQ1       V R G V T R H I Y N R E E Y V|R F D S|C V G V Y R A V T P Q G
       DQ1       V R L V T R Y I Y N R E E Y A|R F D S|D V G V Y R A V T P Q G
HLA    DQ3       V R L V S R S I Y N R E E V V|R F D S|D V G V Y R A V T P Q G
       DQ3       V F L V S R S I Y N R E E I V|R F D S|D V G E F R A V T P Q G
       DQ4       V R L V T R Y I Y N R E E Y A|R F D S|D V G V Y R A V T P Q G
       DR4,w6    V R F L E R H F H N G E E Y A|R F D S|D V G E Y R A V R|E L|G
       DR3,w6    V R L L E R R V H N Q E E Y A|R[Y]D S|D V G E Y R A V T|E L|G
       DR3,w6    V R Y L D R Y F H N Q E E Y A|R F D S|D V G E Y R A V T|E L|G
       DR2       V R F L D R Y F Y N Q E E S V|R F D S|D V G E F R A V T|E L|G
       DP2       V R F L H R D I Y N Q E E D L|R F D S|D V G E Y R A V T|E L|G

Classe II B
           d
       I-Ad     I R L V T R Y I Y N R E E Y V|R[Y]D S|C V G E Y R A V T|E L|G
           k
       I-Ak     I R L V I R Y I Y N R E E Y V|R F D S|D V G E Y R A V T|E L|G
H-2        b
       I-Ab     I R Y V T R Y I Y N R E E Y V|R[Y]D S|D V G E H R A V T|E L|G
           d
       I-Ed     V R F L E R F I Y N R F E N L|R F D S|D V G E Y R A V T|E L|G
           b
       I-Eb     V R L L E R Y F Y N L E E N L|R F D S|D V G E F R A V T|E L|G
           k
       I-Ek     V R L L V R Y F Y N L E E N L|R F D S|D V G E F R A V T|E L|G

       - - - - - - - - - - - - - - - - - - - - - - - - - - - - - - - - - - - - -

       LAV1a     S L H G M D D P E R E V L E W|R F D S|R L A F H H V A R|E L|H
SIDA   HTLVIII   S L H G M D D P E R E V L E W|R F D S|R L A F H H M A R|E L|H
       ARV2      S L H G M E D A E K E V L V W|R F D S|K L A F H H M A R|E L|H
       LV        S L H G M D D P E R E V L E W|R F D S|R L A F H H V A R|E L|H

                 .           .               .
               170         180             190
```

séquences peptidiques communes ont été amenés en regard mutuel.

Dans la partie gauche du tableau sont indiqués les antigènes dont les séquences peptidiques montrées sont rediquées. Les séquences peptidiques communes sont encadrées.

Ces observations sont à la base de l'invention, laquelle a, à son tour, confirmé la validité des hypothèses initialement posées. La synthèse de peptides contenant de telles séquences a confirmé la validité de l'hypothèse intitialement faite. Des peptides contenant la séquence RFDS se sont révélés capables d'adhérer aux lymphocytes T4 et à prévenir l'adhésion du virus LAV sur les lymphocytes ainsi modifiés. Ces peptides se sont ainsi révélés comme constituant l'un des déterminants intervenant dans la reconnaissance mutuelle des lymphocytes et soit des susdits antigènes, soit des virus LAV, d'où l'expression "peptide adhésiotope" qui sera utilisée dans ce qui suit.

A l'instar de ce qui a été observé dans d'autres systèmes qui ont été décrits, par exemple celui qui a conduit à la détermination du site de reconnaissance de nombreuses cellules eucaryotes par la fibronectine (1), (2) et (3) (site qui s'est trouvé être localisé dans une séquence RGDS), on a constaté que dans le cas de l'invention, le peptide RFDS pouvait être remplacé par un peptide ayant une séquence inversée ("forme miroir"), c'est-à-dire le peptide SDFR.

Dans la mesure où le site RFDS est porté par le virus LAV ou par les antigènes MHC spécifiques des lymphocytes T4 (ou d'autres lymphocytes, tels que les lymphocytes T8), il en découlait aussi la possibilité que les molécules T4 des lymphocytes eux-mêmes devaient comporter des séquences peptidiques définissant les sites susceptibles d'intervenir dans la reconnaissance mutuelle susdite. L'invention devait ainsi permettre de reconnaître que cette séquence devait normalement être formée par l'un des peptides RADS ou SDAR, en raison de leur configuration spatiale et des charges portées par certains de leurs résidus aminoacide (charges complémentaires de celles des résidus chargés de RFDS ou SDFR).

Les conditions qui précèdent peuvent être illustrées par le schéma suivant :

```
MHC  .....  R  F  D  S  ....>


             +      -

             -      +

  <.....S  D  A  R  .......  T4
```

L'invention concerne donc plus particulièrement tout peptide contenant au moins une séquence tétrapeptidique choisie parmi RFDS, SDFR, RADS qui constitue un site présent dans la molécule T, ou SDAR, ce peptide présentant une configuration telle que la séquence tétrapeptidique soit exposée dans des conditions lui permettant d'interagir avec un récepteur cellulaire, tenu de conserver ses propriétés d'adhésiotope.

A cet égard on remarquera que la glycoprotéine d'enveloppe des LAV contient également une séquence tétrapeptidique SDAR (dans ARV 2) ou SDAK (dans LAV, LV, HTLV III).

D'une façon générale, les peptides selon l'invention peuvent être choisis parmi ceux qui sont représentés par les formules générales :

X — — — — — — R F D S — — — — — — Z   (I)
X — — — — — — S D F R — — — — — — Z   (II)
X — — — — — — S D A R — — — — — — Z   (III)
X — — — — — — R A D S — — — — — — Z   (IV)
X — — — — — — S D A K — — — — — — Z   (V)

dans lesquelles X est un atome d'hydrogène ou un groupe peptidique avec une extrémité N-terminale et Z est un groupe OH ou un peptide ayant une extrémité C-terminale. ces peptides X et Z ne contrevenant pas, lorsqu'ils sont eux-mêmes constitués par des séquences peptidiques, à la nécessité rappelée plus haut d'autoriser l'exposition de la séquence tétrapeptidique.

Des peptides préférés selon l'invention sont constitués par les tétrapeptides eux-mêmes. Des peptides plus actifs sont ceux qui contiennent dans une même structure l'un des tétrapeptides sus-indiqués. Des peptides intermédiaires préférés sont ceux qui sont constitués par des acides aminés n'influençant pas la morphologie d'ensemble du peptide total. par exemple une chaîne comprenant de 2 à 10 résidus alanine.

D'une façon générale, le choix des groupes peptidiques pouvant être associés aux séquences tétrapeptidiques selon l'invention (où dont l'utilisation devrait être évitée) peut être guidé par l'utilisation de programmes permettant la modélisation des configurations des polypeptides susceptibles d'être obtenus au terme de l'assemblage de séries prédéterminées d'aminoacides. De tels programmes ont notamment été décrits par (14). (15) et (16).

D'autres peptides préférés conformes à l'invention sont ceux qui peuvent être représentés par les formules suivantes (dans lesquelles chaque tiret "-" représente un résidu d'aminoacide) :

0 226 513

$$X \text{——} \underline{R\ F\ D\ S} \text{--------} EL \text{——} Z \quad (VI)$$

$$X \text{——} RE \text{----} \underline{R\ F\ D\ S} \text{----} Z \quad\quad\quad (VII)$$

$$X \text{——} RE \text{----} \underline{R\ F\ D\ S} \text{--------} EL \text{——} Z \quad (VIII)$$

dans lesquelles X et Z ont les significations sus-indiquées.

Des peptides particulièrement préférés contiennent des séquences polypeptidiques choisies parmi :

REVLEWRFDSRLAFHHVAREL (IX)
REVLEWRFDSKLAFHHVAREL (X)
REVLEWRFDSKLAFHHMAREL (XI)

respectivement issues des séquences codables contenues dans les génomes de LAV1a. HTLV III. ARV2 et LV rappelées dans le tableau. Les éléments de séquence soulignés correspondent aux séquences davantage spécifiques qui ont été définies plus haut.

D'une façon générale, le nombre total de résidus d'acides aminés contenus dans les séquences peptidiques selon l'invention n'est pas déterminant. Il sera essentiellement limité par des raisons d'ordre pratique, pour ce qui est de la synthèse chimique. Normalement le nombre total d'acides aminés ne dépassera pas 50. Avantageusement, il est inférieur à 30, voire même à 20.

La capacité des peptides adhésiotopes selon l'invention d'inhiber l'adhésion d'un virus LAV à des lymphocytes T4 peut être appréciée, notamment par la diminution, voire la suppression de la fixation du LAV au récepteur membranaire des lymphocytes T4 après que, soit les lymphocytes (dans le cas de peptides contenant RFDS ou SDFR), soit les virus (dans le cas des peptides contenant SDAR ou RADS) aient été préincubés avec ces peptides.

A cet effet on peut utiliser le virus LAV marqué, par exemple couplé à la fluorescéine isothiocyanate (LAV FITC). La propriété adhésiotope du peptide étudié s'apprécie alors par la réduction ou même la disparition totale de lymphocytes retenant le virus marqué. après séparation éventuelle de l'excédent de virus marqué non fixé sur les lymphocytes.

On peut naturellement avoir recours à toute autre méthode appropriée pour la mesure du pouvoir adhésiotope des peptides à l'étude.

Il va naturellement de soi que certains des aminoacides, dans les structures envisagées peuvent être remplacées par d'autres. Eventuellement dans la séquence RFDS, le résidu F peut être remplacé par un résidu Y. D'une façon générale, on peut avoir recours à toute substitution d'aminoacide par un autre aminoacide isostérique.

Les peptides selon l'invention sont utilisables comme réactifs, par exemple pour détecter dans un fluide biologique. tel que le sérum d'un malade, des anticorps dirigés contre les protéines du virus HIV dirigés contre la région de la protéine contenant la séquence RFDS ou contre la région contenant la séquence SDAR (ou SDAK) de l'enveloppe du virus.

L'invention concerne plus particulièrement ces peptides en tant qu'ils peuvent être utilisés pour la fabrication d'anticorps monoclonaux ou polyclonaux susceptibles de les reconnaître spécifiquement. Ces peptides peuvent être modifiés par l'homme du métier pour qu'ils puissent se fixer sur le site de fixation du LAV sur des cellules T4 sans interférer avec l'activité de la protéine T4 avec le système HLA. Ces peptides peuvent être utilisés dans des kits de diagnostic pour la recherche d'anticorps anti-HIV dans le sérum des malades. Ils peuvent aussi être utilisés pour la détection de protéines virales dans le sérum des malades et aussi à des fins thérapeutiques par administration à des malades dans le but d'empêcher la dissémination de l'infection à de nouvelles cellules ou à des personnes non encore atteintes et ce à des fins préventives, pour empêcher l'infection.

L'invention concerne encore les régions intermédiaires entre les sites RFDS et EL, d'une part, et RE, d'autre part. dans les génomes des différentes formes de virus LAV qui ont été isolés.

Ces régions sont caractérisées par un grand degré de conservation. Il s'agit plus particulièrement des régions
VLEW
RLAFHHVAR
KLAFHHVAR
RLAFHHMAR.

L'invention concerne aussi l'application de ces derniers types de peptides. aux mêmes fins que celles qui ont été indiquées pour les précédents peptides. Ils peuvent le cas échéant être utilisés directement pour sa fabrication d'anticorps monoclonaux ou polyclonaux. ceux-ci étant alors aptes à se fixer sur les protéines T4, in vitro ou in vivo sans interférer avec l'activité de la protéine T4 avec le système HLA.

L'invention concerne encore les compositions immunogènes qui peuvent être préparées en mettant en jeu les peptides selon l'invention. le cas échéant amenés sous les formes appropriées à la manifestation in vivo de cette immunogénicité. d'une part. et la production d'anticorps, de préférence monoclonaux, plus spécifiquement dirigés vers les sites spécifiques de ces peptides. Par "sites spécifiques", on entend plus particulièrement ceux qui ont été indiqués dans ce qui précède, en l'occurrence tant les sites RFDS, SDFR, SDAR. RADS. EL ou, d'ailleurs également. LE. RE ou ER (EL et ER faisant également partie de l'invention), que

6

les sites intermédiaires entre ces groupes peptidiques et plus spécifiquement les peptides intermédiaires entre RFDS et EL, d'une part, RFDS et RE, d'autre part, dans les génomes des virus LAV concernés.

Il a de plus été constaté que la séquence RFDS constitue apparemment le site de reconnaissance utilisable par d'autres types de virus à l'égard des cellules qu'ils sont capables d'infecter. Tel est par exemple le cas du rhinovirus HRV14, dont la protéine VP1 contient également la séquence RFDS. La même généralisation peut se faire au niveau d'autres virus, par exemple le virus d'Epstein-Barr, dont une glycoprotéine contient l'adhésiotope RADS.

D'autres peptides préférés selon l'invention sont caractérisés par la formule suivante :
X − − −JRFDSD − − −Z   (XII)
dans laquelle X et Z ont les significations sus-indiquées et J est A, V ou L, ces peptides comportant de 6 à 20, voire 30 résidus aminoacyle.

Des hexapeptides préférés appartenant à cette catégorie de peptides présentent la formule :
$H_2N$-VRFDSD-OH   (XIII)
ou la formule $H_2N$ − − −LRFDSD − − −OH   (XIV)

Un heptapeptide préféré appartenant à cette catégorie présente la formule :
$H_2N$-LRFDSDL-OH   (XV)
(ci-après encore désignée par la formule LRFDSDL ou par l'abréviation "DR-7").

A cet heptapeptide correspond une séquence contenue dans le domaine β1 de molécules HLA-DR de classe 2.

D'autres peptides préférés conformes à l'invention sont caractérisés par la formule :
X − − −EEBJRFDSDVGE − − −Z   (XVI)
dans laquelle :
X et Y ont les significations sus-indiquées,
B est choisi parmi Y, S, D, F ou N, plus particulièrement Y ou D, et
J est choisi parmi A, V, L et avec F pouvant éventuellement être remplacé par Y.

Des peptides préférés appartenant à cette catégorie de peptides sont constitués par des dodécapeptides de formule :
$H_2N$-EEBJRFDSDVGE-OH   (XVII)
et plus particulièrement :
$H_2$-EEYDRFDSDVGE-OH   (XVIII)
(ou encore désigné ci-après par la formule EEYDRFDSDVGE ou par la désignation "DR12").

D'une façon générale, l'invention concerne tout peptide du même type susceptible d'avoir une séquence essentiellement semblable, telle que susceptible d'être présente dans d'autres molécules HLA DR non identifiées dans le tableau présenté à la fin de cette description.

Parmi ces peptides on mentionnera encore plus particulièrement ceux de formule :
X − − −EEBJRFDSDVGEURAVTEL − − −Y   (XIX)
dans laquelle X et Y ont les significations sus-indiquées et U est Y, F ou H.

Dans tous les peptides qui viennent d'être identifiés, il sera naturellement remarqué que les groupes X et Y (dès lors qu'ils sont différents de $H_2N$- et -OH respectivement) doivent être tels qu'ils ne modifient pas les qualités adhésiotopes de la séquence RFDS (ou RYDS) elle-même.

Comme précédemment évoqué, les peptides susindiqués peuvent être remplacés par ceux qui leur correspondent et dans les antigènes T4 des lymphocytes auxiliaires, les séquences étant présumées intervenir dans la reconnaissance mutuelle des HLA-DR et des lymphocytes T4. A titre d'exemple de tels peptides, on mentionnera celui de formule :
DRADSKL   (XX)
(ci-après désigné par "T4-7"). Ces séquences contenues dans les antigènes T4 sont celles qui sont présumées (ce qui a d'ailleurs été vérifié) intervenir dans les réactions de reconnaissance mutuelle des antigènes HLA (en l'occurrence des molécules HLA-DR dans l'exemple considéré) et des lymphocytes T4. A titre d'exemples de tels peptides, on mentionnera :
- celui de formule :
DRADSKL   (XX)
(ci-après désigné par l'abréviation "T4-7"), ce peptide correspondant au peptide "DR-7" susdit, ou
- celui de formule :
KLNDRADSRRSL   (XXI)
(ci-après désigné par l'abréviation "T4-12"), ce dernier peptide correspondant alors au peptide "DR-12".

L'invention concerne donc également, et de façon plus générale, des peptides contenant la séquence RADS et, entourant celle-ci, des séquences peptidiques n'interférant pas avec la capacité de la séquence RADS à être reconnue par des molécules HLA humaines, plus particulièrement HLA-DR.

L'invention concerne encore plus particulièrement parmi les peptides précédents ceux qui sont capables d'exercer l'effet immunomodulateur dont il a été question dans le préambule de cette description, plus particulièrement à l'égard ds antigènes généralement susceptibles d'induire in vivo des réponses immunitaires médiées par des antigènes HLA de classe II, notamment HLA-DR.

A cet égard, il a été constaté que des peptides conformes à l'invention, notamment les peptides de formules XIII, XIV, XVIII, XX et XXI, étaient capables comme les anticorps monoclonaux spécifiques des molécules HLA-DR et T4, respectivement, d'inhiber in vitro les interactions entre les macrophages et les lymphocytes auxiliaires (lymphocytes T4). d'une part. et entre les lymphocytes T4 et les lymphocytes B, d'autre part. Les propriétés inhibitrices de ces peptides ont été mises en évidence soit individuellement, soit dans des essais de synergie d'inhibition réalisés in vitro dans un système expérimental tout à fait analogue à celui décrit par A. FISHER et al. Eur. J. Immunol. (1986), 16 : 1111-1116. visant à étudier les réponses immunes in vitro de ces lymphocytes à l'égard du même virus de la grippe. si ce n'est que ces essais ont été réalisés avec des doses sub-inhibitrices de peptides complémentaires l'un de l'autre (EEYVRFDSDVGE et KLNDRADSRRSL). en lieu et place des doses sub-inhibitrices d'anticorps anti-HLA-DR et anti-T4 respectivement qui avaient été mises en oeuvre dans ces essais de synergie d'inhibition par FISHER et al.

On rappellera en effet que. dans ce système. l'incubation in vitro. d'une part. de lymphocytes T auxiliaires avec un anticorps anti-T4 en concentration infra inhibitrice. et. d'autre part. de lymphocytes B avec un anticorps anti-HLA-DR en concentration infra-inhibitrice. provoque une inhibition puissante de la production d'anticorps.

Il a ainsi été mis en évidence que le peptide EEYVRFDSDVGE (DR-12) analogue de molécules HLA de classe II DR (strictement des molécules murines IA$_k$) inhibe de façon très significative et reproductible la prolifération spécifique du virus influenza des lymphocytes T ainsi que la production d'anticorps contre ce virus à des concentrations de l'ordre de 10 micro-molaires. Cette inhibition est faible sous l'effet du peptide RFDS seul. Des résultats intermédiaires sont obtenus avec des peptides contenant la séquence RFDS entourée d'acides aminés. de ceux qui, dans les molécules HLA de classe II, entourent la séquence RFDS. C'est ce que l'on a notamment observé avec le peptide "DR-7".

Un peptide de 12 acides aminés contenant la séquence RADS, en particulier le peptide KLNDRADSRRSL a une activité inhibitrice modérée mais significative.

L'effet inhibiteur de ces peptides, analogues de la molécule HLA de classe II se sont révélés tout à fait parallèles à ceux que l'on peut obtenir à l'aide d'anticorps monoclonaux dirigés contre la molécule T4. Ainsi un clone de lymphocytes T spécifique du virus influenza dont la prolifération induite par l'antigène est résistante à l'inhibition par des anticorps-anti T4 est également résistante à l'inhibition par les susdits peptides analogues, alors que l'effet auxiliaire (activation des lymphocytes B) de ce même clone est sensible aussi bien aux anticorps anti T4 qu'à ces peptides. analogues de DR.

Un argument supplémentaire en faveur d'une interaction réelle entre RFDS et RADS résulte d'expériences démontrant une synergie d'inhibition par ces peptides des interactions cellulaires.

On observe, dans les conditions expérimentales décrites par FISHER et al, une inhibition importante de la prolifération ou de la production d'anticorps spécifiques du virus influenza, lorsque des lymphocytes T, après avoir été pré-incubés avec des concentrations infra-inhibitrices du EEYVRFDSDVGE, ont été mis en contact, soit avec des monocytes, soit avec des lymphocytes B, qui avaient également été pré-incubés avec des concentrations infra-inhibitrices du peptide analogue de T4 KLNDRADSRRSL. L'effet de synergie ainsi observé n'est pas obtenu en utilisant d'autres peptides ou éventuellement d'autres anticorps sans rapport avec les molécules T4 et classe II.

La validité de ces résultats est confirmée par le fait que les peptides utilisés n'ont pas d'effet cytotoxique sur les cellules employées. Ils n'inhibent pas des tests fonctionnels des lymphocytes T n'impliquant pas les molécules HLA de classe II. D'autre part, en pré-incubant les peptides avec des anticorps anti-peptides spécifiques, on peut bloquer l'effet inhibiteur dû au peptide.

Cependant, les séquences adjacentes à la structure préservée RFDS, mais qui varient entre molécules HLA DR. DQ et DP, pourraient avoir un rôle dans l'établissement d'une structure tertiaire indispensable à l'interaction avec les molécules T4. En effet les peptides analogues de HLA DR n'ont pas d'effet inhibiteur.

Les peptides selon l'invention peuvent être préparés par les techniques classiques, dans le domaine de la synthèse des peptides. Cette synthèse peut être réalisée en solution homogène ou en phase solide.

Par exemple on aura recours à la technique de synthèse en solution homogène décrite par HOUBENWEYL dans l'ouvrage intitulé "Methode der Organischen Chemie" (Méthode de la chimie organique) édité par E. WUNSCH. vol. 15-I et II. THIEME. Stuttgart 1974.

Cette méthode de synthèse consiste à condenser successivement deux à deux les aminoacyles successifs dans l'ordre requis, ou à condenser des aminoacyles et des fragments préalablement formés et contenant déjà plusieurs résidus aminoacyles dans l'ordre approprié, ou encore plusieurs fragments préalablement ainsi préparés. étant entendu que l'on aura eu soin de protéger au préalable toutes les fonctions réactives portées par ces aminoacyles ou fragments. à l'exception des fonctions amine de l'un et carboxyle de l'autre ou vice versa. qui doivent normalement intervenir dans la formation des liaisons peptidiques, notamment après activation de la fonction carboxyle. selon les méthodes bien connues dans la synthèse des peptides. En variante. on pourra avoir recours à des réactions de couplage mettant en jeu des réactifs de couplage classique. du type carbodiimide, tels que par exemple la 1-éthyl-3-(3-diméthyl-aminopropyl)-carbodiimide. Lorque l'aminoacyle mis en oeuvre possède une fonction acide supplémentaire (notamment dans le cas de l'acide glutamique). ces fonctions seront par exemple protégées par des groupes t-butylester.

Dans le cas de la synthèse progressive. acide aminé par acide aminé, la synthèse débute de préférence par la condensation de l'aminoacide C-terminal avec l'aminoacide qui correspond à l'aminoacyle voisin dans la séquence désirée et ainsi de suite. de proche en proche. jusqu'à l'acide aminé N-terminal. Selon une autre technique préférée de l'invention. on a recours à celle décrite par R.D. MERRIFIELD dans l'article intitulé "Solid phase peptide synthesis" (J. Am. Chem. Soc., 45, 2149-2154).

Pour fabriquer une chaîne peptidique selon le procédé de MERRIFIELD, on a recours à une résine polymère très poreuse, sur laquelle on fixe le premier aminoacide C-terminal de la chaîne. Cet aminoacide est fixé sur la résine par l'intermédiaire de son groupe carboxylique et sa fonction amine est protégée, par exemple par le groupe t-butyloxycarbonyle.

Lorsque le premier aminoacide C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'Aide d'acide trifluoroacétique.

On couple ensuite le deuxième aminoacide qui fournit le second aminoacyle de la séquence recherchée, à partir du résidu aminoacyle C-terminal sur la fonction amine déprotégée du premier aminoacide C-terminal fixé sur la chaîne. De préférence, la fonction carboxyle de ce deuxième aminoacide est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux aminoacides, et dont la fonction amine terminale est protégée. Comme précédemment, on déprotège la fonction amine et on peut ensuite procéder à la fixation du troisième aminoacyle, dans les conditions analogues à celles de l'addition du deuxième aminoacide C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents aminoacides constituant la chaîne peptidique et on détache le peptide de la résine par exemple à l'aide d'acide fluorhydrique.

L'invention concerne également les oligomères hydrosolubles des peptides monomères sus-indiqués. L'oligomérisation peut provoquer un accroissement de l'immunogénicité des peptides monomères selon l'invention. Sans qu'une telle indication chiffrée puisse être considérée comme limitative, on mentionnera néanmoins que ces oligomères peuvent éventuellement contenir de 2 à 25 unités monomères.

Elle concerne plus particulièrement ceux des oligomères dans lesquels les motifs adhésiotopes sont reliés les uns aux autres par des chaînons peptidiques intermédiaires n'interférant pas avec leurs possibilités d'exposition. Ces chaînons sont par exemple constitués par des dipeptides Ala-Ala.

On peut avoir recours, pour réaliser l'oligomérisation, à toute technique de polymérisation couramment utilisée dans le domaine des peptides, cette polymérisation étant conduite jusqu'à l'obtention d'un oligomère ou polymère ontenant le nombre de motifs monomères requis pour l'acquisition de l'immunogénicité désirée.

Une méthode préférée de l'oligomérisation ou de polymérisation du monomère consiste dans la réaction de celui-ci avec un agent de réticulation, tel que le glutaraldéhyde.

On peut également avoir recours à d'autres méthodes d'oligomérisation ou de couplage, par exemple à celle mettant en jeu des couplages successifs d'unités monomères, par l'intermédiaire de leurs fonctions terminales carboxyle et amine en présence d'agents de couplage homo- ou hétéro- bifonctionnels.

On peut également, pour la production de molécules comportant un ou plusieurs motifs nonapeptidiques tels que définis ci-dessus, avoir recours à des techniques du génie génétique mettant en oeuvre des micro-organismes transformés par un acide nucléique déterminé comprenant des séquences nucléotidiques appropriées correspondantes.

L'invention concerne encore les conjugués obtenus par couplage covalent des peptides selon l'invention (ou des susdits oligomères) à des molécules porteuses (naturelles ou synthétiques) physiologiquement acceptables et non toxiques, par l'intermédiaire de groupements réactifs complémentaires respectivement portés par la molécule porteuse et le peptide. Des exemples de groupements appropriés sont illustrés dans ce qui suit :

A titre d'exemples de molécules porteuses ou supports macromoléculaires entrant dans la constitution des conjugués selon l'invention, on mentionnera des protéines naturelles, telles que l'anatoxine tétanique, l'ovalbumine, des sérums albumines, des hémocyanines, etc..

A titre de supports macromoléculaires synthétiques, on mentionnera par exemple des polylysines ou des poly(D-L-alanine)-poly(L-lysine).

La littérature mentionne d'autres types de supports macromoléculaires susceptibles d'être utilisés, lesquels présentent en général un poids moléculaire supérieur à 20.000.

Pour synthétiser les conjugués selon l'invention, on peut avoir recours à des procédés connus en soi, tels que celui décrit par FRANTZ et ROBERTSON dans "Infect. and Immunity", 33, 193-198 (1981), ou celui décrit dans "Applied and Environmental Microbiology", octobre 1981, vol. 42, n° 4, 611-614 par P.E. KAUFFMAN en utilisant le peptide et la molécule porteuse appropriée.

Dans la pratique, on utilisera avantageusement comme agents de couplage les composés suivants, cités à titre non limitatif : aldéhyde glutarique, chloroformiate d'éthyle, carbodiimides hydrosolubles (N-éthyl-N'(3-diméthylamino-propyl) carbodiimide, HCl), diisocyanates, bisdiazobenzidine, di- et trichloro-s-triazines, bromures de cyanogène, benzoquinone, ainsi que les agents de couplage mentionnés dans Scand. J. Immunol., 1978, vol. 8, p.7-23 (AVRAMEAS, TERNYNCK, GUESDON).

On peut avoir recours à tout procédé de couplage faisant intervenir d'une part une ou plusieurs fonctions réactives du peptide et d'autre part, une ou plusieurs fonctions réactives des molécules supports. Avantageusement, il s'agit des fonctions carboxyle et amine, lesquelles peuvent donner lieu à une réaction de couplage en présence d'un agent de couplage du genre de ceux utilisés dans la synthèse des protéines, par exemple le 1-éthyl-3-(3-diméthylaminopropyl)-carbodiimide, le N-hydroxybenzotriazole, etc.. On peut encore avoir recours à la glutaraldéhyde, notamment lorqu'il s'agit de relier entre eux des groupes aminés respectivement portés par le peptide et la molécule support.

Des caractéristiques supplémentaires de l'invention apparaîtront encore au cours de la description qui suit des conditions dans lesquelles des peptides préférés de l'invention ont été obtenus.

EXEMPLE 1 :

Synthèse du Arg-Phe-Asp-Ser-Ala-Ala-Arg-Phe-Asp-Ser

Pour préparer ce peptide, on a recours à la synthèse peptidique mentionnée ci-dessus et on procède comme suit ou de façon équivalente.

Les abréviations utilisées dans le cadre de cette synthèse ont la signification suivante :

BOC : t-butyloxycarbonyle

Asp : acide aspartique

Ala : alanine

Arg : arginine

Ser : serine

Phe : phénylalanine.

On utilise comme aminoacides des N-alpha-aminoacides protégés uniquement avec le groupe t-butyloxycarbonyle (BOC).

Les groupes fonctionnels latéraux sont protégés comme suit :

Arg est protégé par le groupe tosyle.

Asp est protégé par le groupe cyclohexyle.

Ser est protégé par le groupe benzyle.

On distille $CH_2Cl_2$ à partir de $Na_2CO_3$ anhydre avant utilisation.

Il est avantageux d'utiliser un support de résine constitué par un copolymère chlorométhylé de styrène et de divinylbenzène à 1 % (commercialisé par les Laboratoires BIORAD).

La N-alpha-t-butyloxycarbonyl-O-benzyl-sérine est de préférence estérifiée sous forme de son sel de césium (GISIN B.F. (1973) Helv. Chim. Acta 56, 1476).

La synthèse est effectuée dans un synthétiseur automatique du type de celui commercialisé par BECKMAN sous la désignation 990 B.

Chacun des acides aminés entrant dans la constitution du peptide selon l'invention est fixé à la chaîne peptidique déjà formée comme il est indiqué ci-après.

a) On lave trois fois la résine pendant environ 3 minutes avec du chlorure de méthylène pour la mettre en suspension.

b) On lave ensuite une fois pendant environ trois minutes avec de l'acide trifluoroacétique à 40 % pour imprégner la résine.

c) On relave une fois pendant environ 30 minutes avec de l'acide trifluoroacétique pour déprotéger le groupe N-terminal de la chaîne peptidique déjà formée.

d) On lave ensuite deux fois pendant environ 3 minutes vec du chlorure de méthylène pour éliminer l'acide trifluoroacétique.

e) Puis on lave deux fois pendant environ 3 minutes avec de l'alcool isopropylique pour éliminer le chlorure de méthylène.

f) Puis on réimprègne le milieu en lavant 4 fois pendant 3 minutes avec du chlorure de méthylène.

g) On lave trois fois pendant environ 3 minutes avec de la diisopropyléthylamine pour neutraliser la fonction amine, qui se trouvait salifiée sous forme de son sel avec l'acide trifluoroacétique.

h) On lave quatre fois pendant environ 3 minutes avec du chlorure de méthylène pour chasser la diisopropylamine en excès.

i) On ajoute l'acide aminé à fixer sur la chaîne peptidique déjà formée, comme indiqué ci-dessous.

j) On lave ensuite trois fois pendant environ 3 minutes avec du chlorure de méthylène.

k) Puis on chasse le chlorure de méthylène en lavant deux fois pendant environ 3 minutes avec de l'alcool isopropylique.

l) On lave enfin trois fois pendant environ 3 minutes avec du chlorure de méthylène, pour remettre le milieu réactionnel dans son solvant.

En ce qui concerne la fixation de chacun des acides aminés à une chaîne peptidique déjà formée, on a recours à un acide aminé (en excès par rapport à la chaine peptidique déjà formée, cet excès correspondant à environ trois fois la charge de la chaîne peptidique) dont la fonction amine est protégée par le groupe t-butyloxycarbonyle et dont la fonction acide est activée par la dicyclohexylcarbodiimide, l'hydroxybenzotriazole ensuite additionné afin de minimiser les réactions secondaires (ARENDT A., KOLODZIEJZKYK A.M. (1978) Tetrahedron Lett 40, 3867), (MOJSOV S., MITCHELL A. R. (1980) J. Org. Chem. 45, 555).

Après chaque étape d'addition d'un acide aminé, on détermine la quantité d'acide aminé libre qui n'a pas été fixée sur la chaîne peptidique, par le test à la ninhydrine (KAISER E., COLESCOTT R.L. et al. (1976) Anal. Biochem. 34, 595).

A la fin de la synthèse, on élimine tous les groupes protecteurs fixés sur le peptide porté par la résine. On détache le peptide de la résine à l'aide d'acide fluorhydrique anhydre à raison de 10 ml d'HF anhydre par gramme de résine, en présence de cresol (1 g par gramme de résine) à la température d'environ 0°C pendant environ 60 minutes.

Après évaporation de l'acide fluorhydrique, le mélange réactionnel est lavé à l'éther et le peptide est séparé de la résine par extraction à l'aide d'acide acétique à 5 %. Les extraits sont dilués à l'eau et lyophilisés.

Le peptide brut obtenu est soluble dans l'eau. Il est purifié par chromatographie sur une colonne du type de celle commercialisée sous le nom GF 05 (IBF) en utilisant comme phase mobile de l'acide acétique à 5 %. On mesure une absorption à 254 nm. Les différentes fractions du pic principal sont examinées sur le plan de leur homogénéité et rassemblées, puis repurifiées par HPLC (abréviation de l'expression anglaise "High Pressure Liquid Chromatography" ou "Chromatographie en Phase Liquide Haute Pression), chargées en le matériau connu sous la marque PARTISIL ODS2 (Whatman, magnum 9), au moyen d'un gradient de 0 à 70 % d'acétonitrile en présence de 1 pour mille d'acide trifluoroacétique. Les fractions sont collectées et lyophilisées avec un rendement global de 25 %.

La composition en acides aminés, après hydrolyse acide totale, confirme la structure du peptide.

On mesure la pureté par chromatographie liquide sous haute pression en phase inversée sur une colonne du type de celle commercialisée sous le nom μ Bondapack C18 dans laquelle l'éluant est un gradient que l'on fait passer en 20 minutes de la composition 0 % d'acétone - 100 % de tampon phosphate 0,0025 M, pH 2,5 à la composition 60 % d'acétone-40 % tampon phosphate.

EXEMPLE 2 :

Synthèse du peptide de formule RADS

La synthèse est réalisée dans des conditions analogues à celles décrites dans l'exemple 1.
On obtient le peptide avec un rendement de 30 %.

EXEMPLE 3 :

De la même façon, on réalise les synthèses de KLNDRADSRRSL et des polypeptides suivants :
SDFRAARFDS
RADSAASDAR
SDARAARADS.

EXEMPLE 4 :

De la même façon, on réalise les synthèses des polypeptides dont les séquences correspondent à celles des virus ou lymphocytes dont les désignations abrégées apparaissent à gauche de la liste qui suit :

HLA-DR2β   VRFLDRYFYNQEESVRFDSDVGEFRAVTELGRPDA
LAV-F       SLHGMDDPEREVLEWRFDSRLAFHHVARELPHEYF
HRV-14-VP1 SLVQLRKKLELFTYVRFDSEYTILATASQPDSANY
T8          RLGDTFVLTLSDFRRENEGYYFCSAL
T4          NQGSFLTKGPSKLNDRAFSRRSLWDQGNFPL
EBV         DGNKETFHERADSFHVRTNYKIV

EXEMPLE 5 :

Séquence contenue dans la glycorprotéine d'enveloppe
- de ARV2 : GVPVWWKEATTTLFCASDARAYDTEVHNVWAT
- de lav )
HTLV III ) : GVPVWWKEATTTLFCASDAKAYDTEVHNVWAT
LV       )

EXEMPLE 6 :

Les peptides suivants ont été synthétisés (et certains d'entre eux testés, comme décrit plus haut, dans des essais de médiation cellulaire in vitro selon la technique de FISHER et al) :
- Peptides de 4 acides aminés
  "DR-4" : RFDS HLA classe II
- Peptides de 7 acides aminés
  "T$_4$-7" : DRADSRK T$_4$
  "DR-7" : LRFDSDL DR$_2$
- Peptides de 10 acides aminés
  RFDS-A-A-RFDS
  SDFR-A-A-SDFR
- Peptides de 12 acides aminés
  "T$_4$-12" : KLNDRADSRRSL
  "DR-12" : EEYDRFDSDVGE

L'efficacité des peptides selon l'invention, en tant qu'inhibiteurs de l'adhésion du LAV à des lymphocytes T4, est illustrée ci-après pr les résultats qui ont été obenus avec le peptide RFDS AA RFDS.

La fixation du LAV à son récepteur membranaire a été directement appréciée par l'analyse de la fixation membranaire du virus LAV couplé à la fluorescéine (LAV FITC). En bref, $10^6$ cellules sont incubées avec 6 microgrammes de LAV FITC, lavées, puis examinées au microscope à fluorescence. Par cette technique, on détecte habituellement environ 10 % de cellules marquées dans une population de cellules T4 + purifiées. Pour évaluer la capacité du peptide synthétique à bloquer cette fixation, des cellules T4 ont été préincubées avec des concentrations variables du peptide, pendant 30 minutes à 4°. Après lavage de l'excès du peptide non fixé, un marquage classique par le LAV FITC. Par cette technique, on a pu observer que le peptide étudié inhibait complètement la fixation du LAV FITC à son récepteur jusqu'à des concentrations aussi basses que 1 microgramme par millilitre.

On décrit ci-après des conditions préférées dans lesquelles les interactions cellulaires entre les monocytes, les lymphocytes T4 et les lymphocytes B peuvent être influencées par les peptides selon l'invention.

## 1 - Préparation des cellules mononucléées du sang périphérique (CMP)

Le sang veineux est collecté à partir de volontaires sains sur liquémine (2500 U. 1/ml-Roche) et dilué dans une solution de Hank's 1 X (Biomérieux). Les cellules mononucléées sont ensuite isolées par centrifugation sur Ficoll-Hypaque (Pharmacia Uppsala. Suède). densité 1,077. lavées et resuspendues dans du milieu RPMI 1640 (Gibco-Biocult, Ecosse) supplémenté avec 1 ml de L-glutamine (Biomérieux), 10 % d'un pool de sérum AB humain décomplémenté (30 minutes à 56°C) et d'antibiotiques (100 U/ml pénicilline, 100 µg/ml Streptomycine).

## 2 - Enrichissement en lymphocytes T - Rosettes E (17)

### 2-1 Sensiblilisation des globules rouges de mouton

Les globules rouges de mouton sont lavés trois fois en milieu Hank's 1X. 20 ml de suspension de globules rouges à 2% sont sensibilisés avec 1 ml de Neuraminidase 1 U/ml (test. Neuraminidase-Behring Werke-Allemagne) pendant 30 minutes à 37°C. Les globules rouges sensibilisés sont lavés trois fois en Hank's et réajustés à 2%.

### 2.2 Absorption du sérum de veau foetal (SVF)

Le sérum de veau foetal (Gibco) est absorbé trois fois sur globules rouges de mouton (1 volume de culot globulaire pour 1 volume de sérum de veau foetal)
- deux fois 30 minutes à 37°C
- une fois 60 minutes à 4°C

### 2.3 Formation des rosettes E

Les lymphocytes sont mis à une concentration de $10^7$/ml dans du RPMI 1640 sans sérum. 2 ml de suspension de globules rouges sensibilisés à la neuraminidase et 0.2 ml de SVF absorbé sont ajoutés à 1 ml de suspension lymphocytaire.

Les tubes sont centrifugés à 200 g pendant 5 minutes placés dans la glace pendant 1 heure au minimum.

Le culot est ensuite remis en suspension très délicatement. La population cellulaire formant rosettes (E+), très enrichie en lymphocytes T est séparée du reste des cellules (E-) par sédimentation sur gradient de Ficoll à 4°C.

Les globules rouges sont ensuite lysés par un choc hypotonique et les lymphocytes sont lavés trois fois en Hank's.

## 3 - Séparation des populations T, B, monocytes

Les monocytes sont obtenus après adhérence des cellules mononucléées ($10^7$/ml) sur boîte de Pétri (Corning) pendant une heure à 37°C. Ces boîtes de Pétri ont été préalablement incubées à 4°C avec du SVF décomplémenté, durant la nuit. Après avoir récupéré les cellules non adhérentes, les cellules adhérentes sont détachées avec un barreau caoutchouté (rubber policeman) et par un pipettage vigoureux. Après deux lavages, elles sont irradiées à 4000 rads (IBL 137, source $C_S$-137, CEA).

Les monocytes restants sont séparés des cellules non adhérentes par un traitement à la L-méthylleucine ester ($5.10^6$ /ml, 5 mM final, Sigma) 40 minutes à température ambiante (18). La réaction est arrêtée par addition de sérum AB humain (10 % final et lavages à froid).

Les lymphocytes T et B sont séparés par la technique des rosettes E. Les cellules T étant capables de former des rosettes en présence de globules rouges de mouton sensibilisés à la neuraminidase, après une incubation d'une heure à 0°C. Les cellules formant rosettes (E+) seront séparées des cellules ne formant pas rosettes (E- ou B) par sédimentation sur gradient Ficoll-Hypaque. Les globules rouges de mouton sont lysés par choc hypotonique.

## 4 - Obtention de lignées T, interleukine 2 dépendantes, spécifiques du virus influenza

Les lignées spécifiques du virus influenza Bangkok (A/Bangkok) sont obtenues à partir de cellules mononucléées de donneurs sensibilisés (19).

Les lymphocytes sont mis à la concentration de $10^6$ /ml dans du milieu RPMI, contenant 10 % de sérum AB humain (SAB) en présence du virus A/Bangkok à 5 µg/ml. 10 ml sont répartis dans des flacons de culture (25cm² , Corning).

Après 7 jours de culture à 37°C en atmosphère humide contenant 5 % de $CO_2$, les cellules mortes sont

12

éliminées par sédimentation sur gradient de Ficoll. Les blastes sensibilisés par le virus sont recueillis et réajustés à $(10+5)$/ml dans du milieu RPMI, 10 % SAB contenant de l'interleukine 2 recombinant (BIOGEN-Suise) testé sur lignée CTLL$_2$, et utilisé au 1/16000 final, en présence de lymphocytes autologues irradiés à 4000 rads ou de lignées B autologues immortalisées par le virus d'Epstein-Barr, irradiées à 8000 rads (cellules de supplémentation ou "feeders"). Ces "feeders" autologues sont ajoutés tous les 7 jours à un rapport : 1 "cellule feeder" pour 1 cellule de lignée. Le milieu de culture est remplacé par du milieu frais contenant 50 % de surnageant riche en IL2, et de l'IL 2 recombinant, les cellules étant réajustées à $(10+5)$/ml tous les trois jours.

## 5 - Obtention des lignées B immortalisées par le virus d'Epstein-Barr (EBV)

Des cellules mononucléées fraiches ou décongelées sont resuspendues à la concentration de $2.10^6$ /ml dans du millieu RPMI contenant 20 % de sérum de veau foetal décomplémenté, en présence d'EBV, obtenue à partir de lignées hématopoïétiques B95-8 de marmosrt (N.I.G.M.S. Copewood St. Camden), et de cyclosporine A à 1 µg/ml final, bloquant toute activation des lymphocytes T.

La culture se fait à 37° C en atmosphère humide contenant 5 % de $CO_2$. Le milieu est changé tous les 5 jours.

## 6 - Obtention des surnageants riches en IL 2

Des lymphocytes sont isolés du sang de deux ou trois donneurs, après incubation du sang une nuit à 37° C. Ces lymphocytes sont ensuite regroupés, irradiés à 1000 rads et ajustés à $10^6$ ml en RPMI 1640 supplémenté en L-glutamine (2 mM), antibiotiques, 20 % de sérum AB humain décomplémenté et 2 µg/ml de phytohémagglutinine purifiée (DIFCO).

Après 48 heures de culture à 37° C en atmosphère humide contenant 5 % de $CO_2$, le surnageant, riche en IL 2, est recueilli, filtré (filtre de 0,45 µm de porosité, Schlücher et Schell, Allemagne) et congelé à -20° C jusqu'à l'emploi.

L'IL 2 recombinant (BIOGEN, Genève, Suisse) testé sur lignées CTLL 2, et ajouté aux surnageants précédents, est aussi utilisé dans les tests de prolifération cellulaire au 1/16000 final.

## 7 - Test de prolifération cellulaire

La prolifération lymphocytaire est induite soit par des mitogènes :
- Phytohémagglutinine (PHA) 1/700e (DIFCO), soit par des antigènes après sensibilisation du sujet (naturelle ou par vaccination),
- Candidine (Cd) 500 µg/ml (Institut Pasteur),
- Virus influenza A/Bangkok 2 µg/ml (Institut Pasteur).

Les cellules sont incubées à 37° C en atmosphère humide contenant 5 % de $CO_2$ pendant trois jours pour les proliférations induites par la PHA, et de six jours pour les proliférations induites par les antigènes précédents.

Selon le type de cellules utilisées : fraiches ou lignées, le test subira quelques modifications.
- Pour les cellules fraiches, les lymphocytes sont mis à la concentration de $10^6$ /ml dans du RPMI 1640 contenant 10 % de sérum AB humain décomplémenté, supplémenté de L-glutamine et d'antibiotiques, 200 µl de la suspension cellulaire sont distribués dans des microplaques à fond rond, en triple (FLOW).
- Pour les populations séparées, les lymphocytes sont mis à la concentration de $10^6$ /ml, dans le milieu RPMI 10 % sérum humain, et les monocytes irradiés à $0,25.10^6$ /ml, 100 µl de chaque suspension cellulaire sont distribués dans des microplaques à fond rond, en triple.
- Pour les lignées T spécifiques du virus influenza $15.10^3$ cellules de la lignée préalablement lavées pour éliminer le TCGF, sont recombinées avec $3.10^4$ lymphocytes irradiés à 4000 rads, dans des microplaques à fond rond. Pour les lignées, la culture est de 72 heures.

Dans tous les cas, l'induction de la prolifération est réalisée avec 1 à 2 µg/ml de virus influenza A/Bangkok. La prolifération cellulaire est mesurée à l'aide d'un compteur à scintillations liquides (Minaxi tri-carb 4000, Packard) par l'incorporation de thymidine tritiée (CEA), 1 µCi/puits, 8 heures avant la fin de la culture. 4 lignées que nous avons obtenues sont testées : PT3, HT4. PT2, FPT1, ainsi qu'un clone T auxiliaire (helper) disponible, obtenu par dilution limite : SB 12 (8).

## 8 - Test pour la production d'anticorps anti-influenza

Les préparations cellulaires T, B, séparées par la technique des rosettes E, et les monocytes irradiés, sont resuspendus dans du milieu RPMI 1640 contenant 10 % de sérum de chèvre décomplémenté (Flow), recombinés à raison de $10.^5$ lymphocytes B. $5.10^4$ monocytes, $2.10^5$ lymphocytes T qui sont les concentrations optimales préalablement mises au point par le laboratoire (16). Le virus A/Bangkok est ajouté à la concentration finale de 2 µg.

Les cellules sont incubées à 37° C en atmosphère humide contenant 5 % $CO_2$ durant 7 jours. Les plaques sont alors centrifugées et les 200 µl de surnageant sont remplacés par 100 µl de RPMI 1640 supplémenté en L-glutamine (1 mM). antibiotiques et 1 vol. de sérum de veau foetal décomplémenté. Après 5 jours de culture supplémentaires, les surnageants sont récoltés et stockés à -2 C. La détermination des anticorps anti-influenza présents dans les surnageants de culture est réalisée par la méthode immuno-enzymatique (ELISA).

9 - Dosage des anticorps - technique ELISA (20)

Fixation de l'antigène
50 µl de virus influenza (50 µg/ml)
1 heure à 37 C
2 lavages PBS. 1 lavage PBS-BSA 3 %
  PBS : tampon phosphate saline
  BSA : albumine bovine

Saturation des sites de fixation
50 µl de PBS-BSA 3 %
1 heure à 37 C
2 lavages PBS - 1 lavage PBS-BSDA 3 %

Fixation spécifigue des anticorps à doser
50 µl de surnageants à tester
1 heure à 37°C
2 lavages PBS - 1 lavage PBS-BSA 2 %

Fixation de l'antiglobuline couplée à la phosphatase alcaline
50 µl de GaHIgG-phosphatase alcaline
1 heure à 37°C
2 lavages PBS - 1 lavage PBS-BSA 2 %
3 lavages tampon bicarbonate pH 9,6, $MgCl_2$ 1,07 M

Réaction enzyme substrat
100 µl de p-nitrophénylphosphate disodique (1 mg/ml). Lecture de la densité optique à 405 nm sur un spectromètre MULTISKAN TITERTEX (FLOW).

10 - Séquences peptidiques utilisées

- Peptides de 4 acides aminés
"DR-4" : RFDS HLA classe II

- Peptides de 7 acides aminés
"$T_4$-7" : DRADSRK $T_4$
"DR-7" : LRFDSDL $DR_2$

-Peptides de 12 acides aminés
"$T_4$-12" : KLNDRADSRRSL
"DR-12" : EEYDRFDSDVGE

11 - Anticorps monoclonaux utilisés
$OKT_4A$ (IgG 2) Anticorps monoclonaux de la série OKT
$OKT_4D$ (IgG 1) (ortho-diagnostic) préparés chez la souris, révélant les lymphocytes T dits inducteurs.
$IOT_3$ (IgG2a) Anticorps monoclonaux (Immuno Tech)
$IOM_1$ (IgG1) préparés chez la souris et révélés par une antiglobuline anti-immunoglobuline de souris couplée à un fluorochrome (Nordic)
$IOT_3$ révélant l'ensemble des lymphocytes T matures
IOM1 révélant les monocytes
L243 (IgG2a) Anticorps monoclonaux (Becton-Dickinson)
CA206 (IgG2a) révélant les molécules HLA-DR
L243
206
Leu 1 (IgG2a) Anticorps monoclonal révélant les populations T (CD5) (Becton-Dickinson).

RESULTATS

1 - Inhibition de la prolifération des lymphocytes T

1-2 - Utilisation de cellules T fraiches

**Figure 1**

Cette figure est illustrative de la variation de la densité optique (fonction du taux de prolifération) en fonction de la concentration en peptide (en µg/ml) dans le milieu de culture dans l'essai qui suit.

Au cours d'un test de prolifération cellulaire de 6 jours induit par le virus influenza A/Bangkok, on observe une inhibition significative par le peptide de 12 acides aminés ou "DR-12" (66 %). Cette inhibition est dépendante de la dose et reproductible ; elle varie d'individu à individu entre 50 et 70 % pour une concentration de 10 µg/ml de peptide. Elle est très proche de l'inhibition produite par l'anticorps monoclonal OKT4 qui est de 86 %. Un autre peptide de 12 acides aminés : "T$_4$-12" a un effet inhibiteur modéré variable (38 %).

**Figure 2**

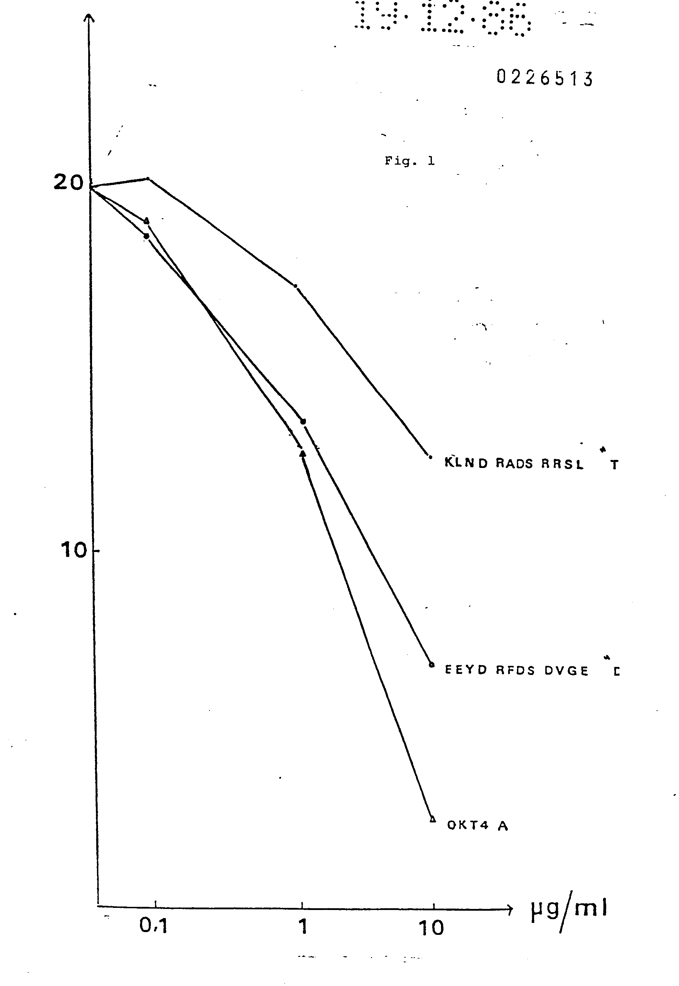

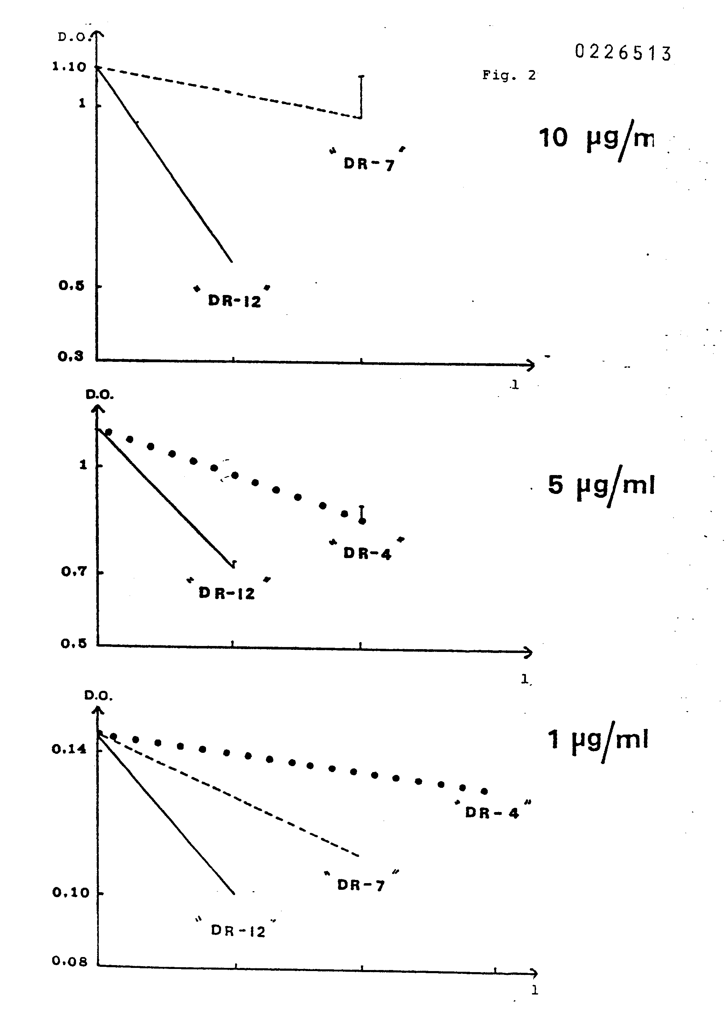

Si on compare l'effet inhibiteur de trois types de peptides "DR" se différenciant par le nombre d'acides aminés entourant la séquence RFDS :"DR-12", "DR-7", "DR-4", on note une corrélation entre la longueur des séquences peptidiques (1 sur l'axe des abscisses de la fig. 2) et l'intensité de l'inhibition (en ordonnées à la fig. 2).

Ces effets sont aussi reproductibles, et peuvent être retrouvés dans d'autres systèmes de prolifération tels le système tuberculine ou tétanos (non représenté).

Il a été vérifié par comptage des cellules au bleu de trypan que cette inhibition par les peptides n'était pas due à un effet cytotoxique. Aucune inhibition n'est par contre observée dans un système induit par des lectines comme la PHA :

|  | cpm |
|---|---|
| PBL seuls | 893 |
| PBL + PHA (1/700) | 47.527 |
| PBL + PHA + OKT$_4$-D (1 µg/ml) | 44.083 |
| PBL + PHA + L243 (0,1 µg/ml) | 40.119 |
| PBL + PHA + "DR-12" (5 µg/ml) | 49.796 |
| PBL + PHA + "DR-12" (10 µg/ml) | 51.570 |

**1-3 Utilisation de lignées T antigène spécifique**

Quatre lignées T auxiliaires (helper), spécifiques du virus A/Bangkok ont été testées avec les peptides de 12 acides aminés : "DR-12" et "T$_4$-12" utilisés à 5 µg/ml final. Une inhibition très significative de 60 % à 90 % est notée par les deux peptides. On trouve une corrélation entre les effets du peptide "DR-12" et de l'anticorps OKT$_4$-A. Cette corrélation d'inhibition est retrouvée avec le clone T helper SB 12 (décrit par FISHER et al) : aucune inhibition n'est observée avec le peptide "DR-12", ni avec l'anticorps OKT$_4$-A. Les résultats apparaissent au tableau 1 ci-après.

**2 - Inhibition de la prolifération des lymphocytes après incubation spécifique avec les peptides analogues**

Afin de préciser le mécanisme de l'inhibition induite par le peptide "DR-12", des expériences

## Tableau 1

### Effet des peptides utilisés sur des lignées T influenza spécifiques

Peptides utilisés à 5 µg/ml

OKT4 A utilisé à 1 µg/ml

| Lignées T | % d'inhibition sur la prolifération influenza | | | % d'inhibition sur la production d'anticorps | | |
|---|---|---|---|---|---|---|
| | "DR-12" | OKT4 A | "T4-12" | "DR-12" | OKT4 A | "T4-12" |
| PT3 | 90 | 85 | 80 | / | / | / |
| MT4 | 80 | 85 | 55 | / | / | / |
| PT2 | 40 | 75 | 75 | / | / | / |
| FPT1 | 60 | 65 | 70 | / | / | / |
| Clone SB12 | 0 | 12 | 0 | 75 | 90 | 20 |

0 % d'inhibition : PT3:6000 cpm, MT4:6300, PT2:5000 FPT1:4500, SB12:15200

## Tableau 2

### Test de fonctionnalité et phénotype des populations séparées

| | CPM |
|---|---|
| T + mγ + PHA | 56.000 |
| T + mγ + IL2 | 55.000 |
| T + mγ + cd | 17.000 |
| T + cd | 2.246 |
| T seuls | 1.000 |
| Monocytes seuls | 184 |

T : lymphocytes T

mγ : monocytes

cd : candidine

d'incubation des populations lymphoïdes purifiées avec les peptides ou des anticorps monoclonaux spécifiques des molécules T4 et HLA-DR ont été effectuées.

**0 226 513**

Il a d'abord été vérifié que les différents traitements utilisés pour séparer les populations lymphoïdes, en particulier la L-leucine méthyl ester, n'affecte pas la fonction des lymphocytes T et leur capacité à proliférer après stimulation par un antigène ou un mitogène. Il a aussi été montré que la population T est suffisamment dépourvue de monocytes pour éviter d'éventuelles interférences dans les incubations. Les résultats résultent du tableau 2.

Sur le modèle des résultats obtenus antérieurement à l'aide d'anticorps anti-T$_4$ (ou T$_4$-D à 1 µg/ml final), on a cherché à mettre éventuellement en évidence une synergie d'inhibition entre peptides équivalents de DR et anticorps anti-T$_4$ ainsi qu'entre peptides équivalents de T$_4$ et anticorps anti-HLA-DR (20 à 10$^{+3}$M final). Les résultats apparaissent dans les tableaux 3 et 4.

L'expérience 1 montre une synergie d'inhibition entre les peptides "DR-12"/"T$_4$-12", "DR-12"/anticorps anti-HLA-DR : 20 ainsi qu'entre les anticorps monoclonaux OKT$_4$D/206 et OKT$_4$D/"T$_4$".

Les mêmes synergies d'inhibitions sont retrouvées dans l'expérience 2 entre "DR-12" et "T$_4$-12" ainsi qu'entre "T$_4$-12" et OKT$_4$D.

Dans cette expérience 2, ainsi que dans l'expérience 3, d'autres peptides de longueur différente ont été testés :

## Tableau 3

### Inhibition de la prolifération des lymphocytes T après incubations spécifiques avec les peptides équivalents de T4 et de DR

| Populations incubées | | Pourcentages d'inhibitions | |
|---|---|---|---|
| T | Monocytes | Expérience 1 | Expérience 2 |
| Peptides | utilisés | 0 % 9300 cpm<br>100% 2000 cpm | 0 % 7600 cpm<br>100% 500 cpm |
| O | 206 | 10 | / |
| DR-12 | O | O | O |
| DR-12 | 206 | 57 | / |
| OKT4D | O | 5 | 15 |
| OKT4D | 206 | 75 | / |
| O | T4-12 | O | 12 |
| DR-12 | T4-12 | 48 | 55 |
| OKT4D | T4-12 | 58 | 45 |
| DR-12 | DR-12 | 60 | / |
| O | DR-12 | 20 | / |
| OKT4D | DR-12 | 60 | / |

Peptides utilisés à 1 µg/ml final

## Tableau 4

### Inhibition de la prolifération des lymphocytes T après incubations spécifiques avec les peptides équivalents de T4 et de DR

| Populations incubées | | Pourcentages d'inhibitions | |
|---|---|---|---|
| T | Monocytes | Expérience 2 | Expérience 3 |
| Peptides | utilisés | 0 % 7600 cpm | 0 % 4000 cpm |
| | | 100% 500 cpm | 100% 200 cpm |
| DR-4 | O | O | O |
| DR-4 | T4-12 | O | 20 |
| DR-7 (0,1) | O | / | 10 |
| DR-7 | O | / | 25 |
| DR-7 (0,1) | T4-12 | / | 40 |
| DR-7 | T4-12 | / | 65 |
| DR-12 | O | O | O |
| O | T4-12 | 12 | 10 |
| DR-12 | T4-12 | 55 | 90 |
| RGDS | O | O | / |
| RGDS | T4-12 | O | / |
| Leu 1 | T4-12 | 5 | / |
| Leu 1 | DR-12 | O | / |

peptides utilisés à 1 µg/ml

"DR-7", "DR-4". De nouveau, aucune action inhibitrice de "DR-4" n'est observée et une action intermédiaire entre "DR-4" et "DR-12" est notée pour le peptide "DR-7" quand il est utilisé en synergie avec "T4-12".

Le test avec l'anticorps Leu 1 (à 1 µg/ml final) montre que les inhibitions ne sont pas dues à un effet non spécifique lié à une simple incubation.

Cependant, il est à noter l'inhibition obervée lors de l'incubation des monocytes avec le peptide "DR-12" utilisé synergie avec OKT4-D ou avec "DR-12".

3 - Inhibition de la réponse anticorps

Nous avons testé si ces peptides "DR" pouvaient bloquer la fonction auxiliaire (helper) des lymphocytes T vis-à-vis des cellules B. Après séparation par rosettes E, les populations sont recombinées à raison de $2.10^5$ E+, $1,5.10^5$ E- en présence des différents peptides : "DR-12", "DR-4", leurs effets étant comparés à celui de l'anticorps monoclonal OKT4-A. Ce test sera fait à trois concentrations finales : 0,1 µg/ml, 1 µg/ml, 10 µg/ml - Figure 4.

Les effets sont tout à fait comparables à ceux obtenus lors des tests de prolifération antigène spécifique. De même, quand on compare les effets des peptides de différentes longueurs - Figures 5-6-7. L'inhibition la plus élevée a lieu avec le peptide "DR-12", puis avec le peptide "DR-7". Une faible, voire aucune inhibition, n'est observée avec le peptide "DR-4".

4 - Inhibition de la réponse anticorps après incubation spécifiques avec les peptides analogues

Le même type d'expériences a été effectué dans le cas de la réponse anticorps in vitro en incubant les lymphocytes T avec l'anticorps OKT4-A (0,1 µg/ml ou 1 µg/ml final) ou avec le peptide "DR-12" (1 µg/ml ou 5 µg/ml final) : les lymphocytes B sont incubés avec le peptide "T4-12" (1 µg/ml ou 5 µg/ml final ) - Figure 8.

Parallèlement aux tests de prolifération, on note une synergie d'inhibition entre "DR-12" et "T₄-12" et entre OKT₄A et "T₄-12". On note donc une corrélation entre "DR-12" et OKT₄A. L'effet est là aussi dépendant de la dose et reproductible.

Les résultats des expériences d'inhibition de la prolifération des lymphocytes T induite par le virus influenza et de la production d'anticorps spécifiques HLA-DR restreints vis-à-vis du même virus, par différents peptides analogues de HLA et de T₄ peuvent se résumer ainsi :

- Deux peptides respectivement de 12 et 7 acides aminés analogues de DR et comprenant la séquence RFDS, inhibent, de façon significative et dépendante de la concentration, la prolifération et l'effet helper T spécifique du virus influenza.

- L'inhibition obtenue est parallèle à celle consécutive à l'addition d'anticorps anti-T₄. Il est en particulier intéressant d'observer que la prolifération spécifique d'un clone T (SB 12) HLA-DR restreint, n'est ni inhibée par les anticorps T₄ ni par le peptide "DR-12", alors que l'effet helper pour la production d'anticorps anti-influenza de ce clone est inhibé à la fois par les anticorps anti-T₄ et le peptide "DR-12".

- Il existe un effet modéré et variable d'inhibition de la prolifération T et de la réponse anticorps anti-influenza par le peptide de 12 acides aminés analogue de T₄ incluant la séquence RADS.

- Il semble exister une synergie d'inhibition de la prolifération T et de la production d'anticorps lorsque les lymphocytes T sont préincubés avec le peptide "DR-12" ou un anticorps anti-T₄, et les monocytes ou les lymphocytes B avec le peptide "T₄-12" ou un anticorps antiHLA-DR.

Plusieurs hypothèses peuvent expliquer l'effet inhibiteur des peptides "DR-12" et "DR-7" :

- Ces peptides se fixent effectivement sur la molécule T₄ à la surface des lymphocytes T et inhibent de fait l'interaction T₄-classe II nécessaire à l'activation et indispensable lors de la coopération T-B.

En faveur de cette hypothèse, on peut retenir la bonne corrélation entre le degré d'inhibition et le caractère HLA-DR restreint de la réponse immune testée.

Le fait que la prolifération spécifique du clone SB n'est inhibée ni par le peptide "DR-12", ni par l'anticorps anti-T₄, alors qu'une inhibition est observée dans la fonction helper par anti-T₄ et le peptide "DR-12" indique également l'existence d'un étroit parallélisme entre les deux inhibitions.

Les réactions qui précèdent permettent également l'étude de la capacité de substances déterminées à exercer une action immunomodulatrice que ce soit au niveau des lymphocytes T4 ou de celui des molécules HLA et à détecter si l'interaction, induite par un antigène déterminé, entre les différents types de cellules qui ont été étudiées, relève d'un type déterminé de molécules HLA, par exemple HLA-DR.

Par exemple dans le cas d'une molécule déterminée, dont aurait été constatée la capacité d'inhiber les interactions, causées par un antigène déterminé, entre monocytes et lymphocytes T, d'une part, les lymphocytes T et lymphocytes B, d'autre part, lorsque cette molécule déterminée as été utilisée à une dose suivante, il sera ensuite possible, notamment en la substituant à l'un ou l'autre des peptides complémentaires dans les essais du type de ceux décrits par FISHER qui ont été décrits plus haut, de détecter si son mode d'action est ou non semblable à celui du peptide remplacé.

En variante, les peptides selon l'invention peuvent également permettre de détecter si les réactions immunitaires induites par un antigène déterminé relèvent d'une intervention ou non demolécules HLA de classe II déterminées, notamment de molécules HLA-DR. A cet effet les tests qui ont été décrits plus haut pourront être mis en oeuvre, si ce n'est que le virus influenza sera remplacé, dans les étapes où celui-ci était amené à intervenir, par cet antigène déterminé. Son mode d'action, mettant en jeu ou non les molécules HLA-DR, dépendra de l'observation ou non d'une inhibition des coopérations cellulaires qui auront été constatées.

Comme il va de soi et comme il résulte d'ailleurs déjà de ce qui précède, l'invention ne se limite nullement à ceux de ses modes d'application et de réalisation qui ont été plus spécialement envisagés ; elle en embrasse au contraire toutes les variantes.

Il est entendu que toute la littérature technique à laquelle renvoie la bibliographie incluse dans cette demande, notamment celle qui suit doit être considérée come faisant partie de la présente description, en ce qu'elle serait nécessaire à l'illustration des points à partir desquels la présente invention a été dégagée.

BIBLIOGRAPHIE

(1) Yamada K.M., Ann. Rev. Biochem. 52:761, 1983.
(2) Boucaut J.C. et al, Nature 307:364, 1984.
(3) Rovasio R.A. et al, J. Cell. Biol 96:462, 1982.
(4) Pierschbacher M.D. et al, Cell 26:259, 1981.
(5) Pierschbacher M.D. et al, J. Biol. Chem. 257:9593, 1982.
(6) Pierschbacher M. et al, Proc. Natl. Acad. Sci. USA, 80:1224, 1983.
(7) Pierschbacher M.D. et al, Nature 309:30, 1984.
(8) Yamada K.M. et al, J. Cell. Biol., in press, 1985.
(9) Boucaut J.D. et al, J. Cell. Biol. 99:1822, 1984.
(10) Pierschbacher M.D. et al, Proc. Natl. Acad. Sci. USA 81:5985, 1984.
(11) Cohn M., Cell 33:657, 1983.
(12) Reinherz E.L. et al,. Cell 19:821, 1980.
(13) Sanchez-Madrid F. et al, Proc. Natl. Acad. Sci. USA 79:7489, 1982.
(14) Nowotny J. et al. Nucl. Acids Res. 12:243, 1984.

(15) Brooks B.R. et al, J. Comput. Chem. 4:274, 1983.
(16) Lesk A.M. et al, Science 216:539, 1982.
(17) Malissen M. et al, Proc. Natl. Acad. Sci. USA 79:893, 1982.
(18) Larhammar D. et al. Hum. Immunol. 8:95, 1983.
(19) Mengle-Gaw L. et al. J.Exp. Med. 160:1184. 1984.
(20) Wain-Hobson S. et al, Cell 40:9, 1985.
(21) Muesing M.A. et al. Nature 313:450. 1985.
(22) Ratner L. et al, Nature 313:277, 1985.
(23) Sanchez-Pescador R. et al. Science 227:484. 1985.
(24) Dalgleish A.G. et al. Nature 321:763. 1984.
(25) Klatzmann D. et al.. Nature 312:767. 1984.
(26) Auffray C. et al; Adv. Hum. Genet., in press. 1985.
(27) Steinmetz M. et al. Science 222:727. 1983.
(28) Steinmetz M. et al. Cell 25:683. 1981.
(29) Morita T. et al, Immunogenetics 21:367, 1985
(30) Mellor A.L. et al, Cell 36:139, 1984.
(31) Marche P.N. et al, Immunogenetics 21:71, 1985.
(32) Nairn R. et al, Biochemistry 20:4739, 1981.

## Revendications

1 - Peptide choisi parmi ceux qui sont représentés par les formules générales :

X — — — — — R F D S — — — — — Z
X — — — — — S D F R — — — — — Z
X — — — — — S D A R — — — — — Z
X — — — — — R A D S — — — — — Z
X — — — — — S D A K — — — — — Z

dans lesquelles X est un atome d'hydrogène ou un groupe peptidique avec une extrémité N-terminale et Z est un groupe OH ou un peptide ayant une extrémité C-terminale, ces peptides X et Z ne contrevenant pas, lorsqu'ils sont eux-mêmes constitués par des séquences peptidiques, à la nécessité d'autoriser l'exposition de la séquence tétrapeptidique interne.

2 - Peptide selon la revendication 1, caractérisé en ce qu'il contient dans une même structure l'un des tétrapeptides sus-indiqués et le tétrapeptide "miroir" correspondant, les deux tétrapeptides étant connectés entre eux par un groupe peptidique intermédiaire distinct n'interférant pas avec la possibilité d'exposition de chacun des deux tétrapeptides.

3 - Peptide selon la revendication 2, caractérisé par une chaîne comprenant de 2 à 10 résidus alanine.

4 - Peptide selon la revendication 1, caractérisé par les formules :

X ——— R F D S --------- EL ——— Z

X ——— RE ---- R F D S ——— Z

X ——— RE ---- R F D S --------- EL ——— Z

dans lesquelles chaque tiret "-" représente un résidu d'aminoacide et X et Z ont les significations sus-indiquées.

5 - Peptide choisi parmi :

REVLEWRFDSRLAFHHVAREL REVLEWRFDSKLAFHHVAREL REVLEWRFDSKLAFHHMAREL.

6 - Peptide selon l'une quelconque des revendications 1 à 5, caractérisé en ce que le nombre total d'acides aminés qu'il contient ne dépasse pas 50, avantageusement est inférieur à 30, voire même à 20.

7 - Peptide selon la revendication 1 de formule :

X — — —JRFDSD— — —Z (XII)

dans laquelle X et Z ont les significations sus-indiquées et J est A, V ou L, ces peptides comportant de 6 à 20, voire 30 résidus aminoacyle.

8 - Peptide selon la revendication 7 caractérisé en ce qu'il est choisi parmi :

VRFDSD
LRFDSDL

9 - Peptide selon la revendication 1 de formule :

X — — —EEBJRFDSDVGE— — —Z (XVI)

dans laquelle :

X et Y ont les significations sus-indiquées,

B est choisi parmi Y, S, D, F ou N, plus particulièrement Y ou D, et

J est choisi parmi A, V, L et

avec F pouvant éventuellement être remplacé par Y, ces peptides comprenant de 12 à 20, voire 30 résidus aminoacyle.

10 - Peptide selon la revendication 9, caractérisé en ce qu'il consiste en un dodecapeptide.

11 - Peptide selon la revendication 10, caractérisé par la formule :

EEBJRFDSVGE

dans laquelle B a la signification sus-indiquée.

12 - Peptide selon la revendication 10 dans lequel B est Y.

13 - Peptide caractérisé par la présence d'une séquence RADS, qui peut comporter jusqu'à 20, voire 30 groupes résidus aminoacyle, et comportant de part et d'autre de la séquence RADS des séquences peptidiques n'interférant pas avec la capacité de la séquence RADS à être reconnue par des molécules HLA humaines, plus particulièrement HLA-DR.

14 - Peptide choisi parmi :

RFDSAASDFR
SDFRAARFDS
RADSAASDAR
SDARAARADS.

15 - Anticorps de préférence monoclonaux, caractérisés par leur capacité de reconnaissance spécifique de l'un des peptides selon l'une quelconque des revendications 1 à 14.

16 - Procédé d'inhibition de l'adhésion de virus LAV à des lymphocytes T4 comprenant la mise en contact soit des virus, soit des lymphocytes, avec un peptide conforme à l'une quelconque des revendications 1 à 15.

17 - Procédé pour détecter dans un fluide biologique, par exemple le sérum d'un malade, des anticorps dirigés contre la région du génome du virus LAV qui contient la séquence RFDS, qui comprend la mise en contact de ce fluide biologique avec le virus dans des conditions propres à permettre la réaction immunologique sous-tendant la détection du virus.

18 - Peptide choisi parmi l'un des suivants :

VLEW
RLAFHHVAR
KLAFHHVAR
RLAFHHMAR.

19 - Anticorps monoclonaux spécifiquement dirigés contre l'un des peptides de la revendication 18.

0226513

Fig. 1

KLND RADS RRSL T

EEYD RFDS DVGE C

OKT4 A

20

10

0,1          1          10          µg/ml

10 µg/m

5 µg/ml

1 µg/ml

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 102, 1985, page 431, résumé no. 76884m, Columbus, Ohio, US; A. BERNA et al.: "Immunodetection of a non-structural protein of alfalfa mosaic virus (P2) in infected tobacco plants", & ANN. VIROL. 1984, 135E(3), 285-96 * Résumé * | 1-4,6 | C 07 K 5/00<br>C 07 K 7/00<br>A 61 K 37/64<br>G 01 N 33/569<br>C 12 P 21/02 |
| | --- | | |
| A | WO-A-8 403 506 (GEYSEN) * En entier * | 1,15, 19 | |
| | --- | | |
| A | EP-A-0 067 425 (R. GEDEON) * Page de garde; pages 2-26,45-46 * | 1 | |
| | ----- | | **DOMAINES TECHNIQUES RECHERCHES (Int. Cl.4)**<br><br>C 07 K 5/00<br>C 07 K 7/00<br>A 61 K 37/00<br>G 01 N 33/00<br>C 12 P 21/00 |

Le présent rapport de recherche a été établi pour toutes les revendications

| Lieu de la recherche | Date d'achèvement de la recherche | Examinateur |
|---|---|---|
| LA HAYE | 12-03-1987 | RAJIC M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82